# EUROPÄISCHE PATENTSCHRIFT

(11) **EP 4 051 161 B1**
(45) Veröffentlichungstag und Bekanntmachung des Hinweises auf die Patenterteilung: **23.04.2025**
(21) Anmeldenummer: 20797456.9
(22) Anmeldetag: 26.10.2020
(51) Int. Cl.: A61B 50/30, A61B 50/33, A61L 2/26, A61B 50/34, B65D 21/02, A61B 50/00

(54) **STERILBEHÄLTER ZUR AUFNAHME VON MEDIZINISCHEM STERILGUT**
STERILISATION CONTAINER FOR RECEIVING STERILE MEDICAL GOODS
RÉCIPIENT DE STÉRILISATION POUR LA RÉCEPTION DE PRODUITS MÉDICAUX STÉRILES

(30) Priorität: 28.10.2019 DE 102019129061
(43) Veröffentlichungstag der Anmeldung: 07.09.2022
(73) Patentinhaber: Aesculap AG, 78532 Tuttlingen (DE)
(72) Erfinder: GÖRZ, Dennis, 78532 Tuttlingen (DE); ROSIN, Bianca, 78532 Tuttlingen (DE); STREIT, Eva, 78351 Bodman-Ludwigshafen (DE); KNITTEL, Timo, 78573 Wurmlingen (DE); BAUER, Stephan, 78576 Emmingen (DE); MOTZ, Corvin, 88630 Pfullendorf (DE); REUTER, Michael, 88637 Leibertingen-Thalheim (DE)
(74) Vertreter: Winter, Brandl - Partnerschaft mbB
(86) Internationale Anmeldenummer: PCT/EP2020/080009
(87) Internationale Veröffentlichungsnummer: WO 2021/083827

(56) Entgegenhaltungen:
- EP-A2- 3 434 611
- WO-A1-2019/197494
- WO-A1-2020/169778
- DE-A1- 102010 050 919
- DE-A1- 102018 104 942
- US-A1- 2007 212 277

## Beschreibung

Die vorliegende Erfindung betrifft einen Sterilbehälter zur Aufnahme von gereinigtem / sterilisiertem oder zu reinigendem / zu sterilisierendem medizinischem Gut, mit einem einen Aufnahmeraum für das Gut ausbildenden Sterilbehälterunterteil und einem an dem Sterilbehälterunterteil lösbar anordbaren Sterilbehälterdeckel zum Verschließen des Aufnahmeraums, wobei am Sterilbehälterunterteil zumindest ein Griffelement angeordnet ist, das zwischen einer vom Sterilbehälterdeckel abragenden Trageposition und einer den Sterilbehälterdeckel mit dem Sterilbehälterunterteil verriegelnden Verriegelungsposition positionierbar ist, wobei in dem Sterilbehälterdeckel ein Aufnahmebereich zur vollständigen Aufnahme des Griffelements darin in der Verriegelungsposition ausgebildet ist. Die Erfindung betrifft außerdem ein Verfahren zur Herstellung eines solchen Sterilbehälters.

Sterilbehälter zum Aufnehmen von zu sterilisierendem Gut, wie medizinischen Instrumenten und Geräten, sind aus dem Stand der Technik bekannt. Solche Sterilbehälter werden auch als Siebschale oder Siebtray bezeichnet und finden in Vorrichtungen zum Sterilisieren von medizinischen Geräten Einsatz. Die mit gereinigten medizinischen Geräten, wie Bohrern, Skalpellen, Spiegeln, Kathetern oder ähnlichen Vorrichtungen bestückten Sterilbehälter werden in ein Sterilbarrieresystem, zum Beispiel einen Sterilcontainer, eingesetzt und zu Sterilisierungsanlagen verbrachten, in denen die medizinischen Geräte, während Sie in der Siebschale liegen, sterilisiert werden.

Es sind Sterilbehälter in Form von Siebschalenbehältern bekannt, die ein Siebschalenunterteil, das einen Aufnahmebereich für zu sterilisierendes Gut ausbildet, und einen Sterilbehälterdeckel zum Verschließen des Aufnahmebereichs des Siebschalenunterteils besitzen, um z.B. während eines Reinigungs- oder Waschprozesses zu verhindern, dass leichte oder kleine Instrumente aus dem Aufnahmebereich herausgeschleudert werden, um ein Herausfallen von Instrumenten während eines Transports in Sterilbarrieresystemen, auf Transportwagen, etc. zu verhindern sowie um einen Abstand zwischen Instrumenten und einer Weichverpackung für die Siebschale zu gewährleisten, sodass diese nicht beschädigt werden kann. Das Siebschalenunterteil und der Sterilbehälterdeckel sind gitterartig oder perforiert ausgebildet, um einen Zufluss und Abfluss eines Reinigungs-, Desinfektions- oder Sterilisationsmediums sicherzustellen.

Um eine Handhabung von geschlossenen Siebschalen zu erleichtern, besitzen diese Tragegriffe, die entweder am Siebschalenunterteil oder an dem damit verbundenen Siebschalendeckel angeordnet sind. Außerdem ist zum Verhindern eines unbeabsichtigten Öffnens des Siebschalendeckels bekannt, am Siebschalenunterteil eine Verriegelungsvorrichtung anzuordnen, mit der der Siebschalendeckel mit dem Siebschalenunterteil verriegelt werden kann. Bei einigen bekannten Siebschalen ist eine solche Verriegelungsfunktion in die Tragegriffe integriert. Trotz des Deckels müssen derartige Tragegriffe / Verriegelungsvorrichtungen für einen Nutzer der Siebschale gut erreichbar sein. Besonders wichtig ist, dass die Siebschale vor allem im beladenen Zustand mit gereinigten Instrumenten sicher getragen werden kann. Schließlich ist es zu Transport- und Lagerzwecken erforderlich, dass mehrere Siebschalenbehälter im verschlossenen Zustand, also mit Deckeln, sicher und stabil gestapelt werden können.

Aus der DE 10 2010 050 919 A1 ist ein Siebschalenbehälter zum Aufnehmen von zu sterilisierendem Gut bekannt, mit einem einen Gutaufnahmebereich definierenden Aufnahmebehälter (Siebschalenunterteil) aus gitterartig perforiertem Material, der auf einer Seite von einem ebenen, öffnenbaren Deckel (Siebschalendeckel) aus gitterartig perforiertem Material verschlossen ist. Am Aufnahmebehälter ist zumindest eine Verriegelungsvorrichtung vorhanden, die den Deckel am Aufnahmebehälter in einer geschlossenen Stellung des Deckels festlegt, wobei die Verriegelungsvorrichtung über ein am Deckel angebrachtes Arretierelement hinweg, unter Ausnutzung der Federeigenschaften der Verriegelungsvorrichtung und des Arretierelements, in eine den Deckel verriegelnde Position verschwenkbar ist. Die Verriegelungsvorrichtung besteht dabei aus einem bügelförmigen Griff, der über den eben ausgebildeten Deckel geschwenkt werden kann und in der Verriegelungsstellung auf der Deckeloberseite aufliegt und daher von der Deckeloberfläche hervorsteht. Es ist daher bei dieser Siebschale von Nachteil, dass ein stabiles und lagesicheres Stapeln mehrerer Siebschalen infolge des über die Deckeloberfläche hervorstehenden bügelförmigen Griffelements nicht möglich ist, da keine ebene Standfläche für einen oberen Sterilbehälter zur Verfügung steht.

Aus der US 8,668,111 B2 ist eine Siebschale mit einem Siebschalenunterteil und einem Siebschalendeckel bekannt, der ein durch gegenüberliegende Seitenkanten und gegenüberliegende Endkanten begrenztes Hauptpanel / Deckelfläche besitzt. Der Siebschalendeckel bildet mittels von der Oberfläche des Hauptpanels nach oben vorstehenden Rippen einen Aufnahmebereich für am Siebschalenunterteil schwenkbar angeordnete Verriegelungsgriffe aus, die in einer Funktionsstellung ein nutzerseitiges Tragen des Siebschalenunterteils und in einer anderen Funktionsstellung ein Verriegeln des Siebschalenunterteils mit dem Siebschalendeckel ermöglichen, wobei die Rippen mit den Verriegelungsgriffen eine Schnappverbindung eingehen. Bei dieser Siebschale ist von Nachteil, dass die Griffe des Behälterbodens auf der Hauptebene des Deckels aufliegen, so dass ein stabiles Stapeln mehrerer Siebschalen durch eine aufwändige Deckel- und Bodenstruktur mit weiteren von der Oberfläche des Deckels nach oben vorstehenden Auflagerrippen realisiert ist. Außerdem würden sich die dargestellten Geometrien nur schwer und mit hohem fertigungstechnischem Aufwand aus rostfreiem Edelstahlblech herstellen lassen.

Aus dem Stand der Technik ist eine weitere Siebschale bekannt, bei der zwei auf einander gegenüberliegenden Seiten des Siebschalenunterteils angeordnete Siebschalengriffe linear vertikal verschieblich innerhalb des Aufnahmeraums eingebracht sind. Die Siebschalengriffe sind jeweils zwischen einer herausgezogenen Trageposition und einer in den Aufnahmeraum eingebrachten Ruheposition positionierbar. Zum Verriegeln des Deckels mit dem Unterteil sind separate Verschlusselemente vorgesehen. Bei dieser Siebschale ist von Nachteil, dass die Siebschalengriffe schlecht erreichbar sind.

Es sind Siebschalen bekannt, bei denen ein Tragegriff nicht am Siebschalenunterteil, sondern an dem damit verriegelten Siebschalendeckel angeordnet ist. Auf dem Deckel angeordnete Tragegriffe verhindern eine sichere und stabile Stapelbarkeit von verschlossenen Siebschalen. Um dies zu verhindern sind Siebschalen bekannt, bei denen am Deckel angeordnete Tragegriffe in eine in den Deckel eingebrachte Mulde versenkbar sind. Allerdings besteht dabei weiter der Nachteil eines unsicheren Transports, da dabei das gesamte Gewicht der Siebschale am Deckel getragen wird und es zu einem unbeabsichtigten Öffnen trotz Verriegelung kommen kann.

Eine bekannte Lösung, um bei auf dem Siebschalendeckel angeordneten Tragegriffen oder Verriegelungselementen eine stabile Stapeleignung zu bewirken, ist das Vorsehen von einen Beabstandung bewirkenden Standfüßen an der Unterseite des Siebschalenbehälters. Solche Siebschalen mit Standfüßen sind jedoch in nachteiliger Weise ungeeignet für Weichverpackungen, da diese durch die vorstehenden Füße perforiert und beschädigt werden können.

Des Weiteren ergibt sich ebenfalls aus der DE 10 2018 130 542 A1, der DE 201 05 328 U1, der US 5 065 885 A, der US 2007 / 212 277 A1, der WO 2019 / 197 494 A1, der EP 3 434 611 A2, der DE 10 2018 104 942 A1, der WO 2020 / 169 778 A1 und der US 6 012 577 A relevanter Stand der Technik.

Vor diesem Hintergrund liegt der vorliegenden Erfindung die Aufgabe zugrunde, die genannten Nachteile des Stands der Technik zu verringern, insbesondere einen Sterilbehälter mit Deckel zu ermöglichen, bei der Griffelemente, die ein insbesondere verlier- und lösesicheres Tragen des insbesondere befüllten und damit schweren Sterilbehälters ermöglichen, für einen Nutzer leicht zu erreichen und zu bedienen sind, das Sterilbehälterunterteil mittels der Griffelemente (und nicht am Deckel) getragen werden kann und ein stabiles Stapeln mehrerer verschlossener Sterilbehälter mit Deckel möglich ist, wobei außerdem eine einfache und sichere Verriegelungsfunktion von Unterteil und Deckel bewirkt werden kann.

Diese Aufgabe wird nach der vorliegenden Erfindung gelöst durch einen Sterilbehälter nach Anspruch 1, also durch einen Sterilbehälter mit einem einen Aufnahmeraum ausbildenden Sterilbehälterunterteil und einem an dem Sterilbehälterunterteil lösbar anordbaren Sterilbehälterdeckel zum Verschließen des Aufnahmeraums, wobei am Sterilbehälterunterteil zumindest ein Griffelement angeordnet ist, das zwischen einer vom Sterilbehälterdeckel abragenden Trageposition und einer den Sterilbehälterdeckel mit dem Sterilbehälterunterteil verriegelnden Verriegelungsposition positionierbar ist, wobei in dem Sterilbehälterdeckel ein Aufnahmebereich, insbesondere ein randseitiger Aufnahmebereich, zur Aufnahme des Griffelements darin in der Verriegelungsposition ausgebildet ist, wobei der Aufnahmebereich als Griffmulde in Form einer gegenüber der Deckelebene in den Aufnahmeraum hineinragenden Vertiefung zur vollständigen Aufnahme des Griffelements darin ausgebildet ist, und wodurch sich mindestens ein Steg beziehungsweise Übergang zwischen dem Sterilbehälterdeckel und dem Aufnahmebereich als Griffmulde bildet, der zumindest abschnittsweise fluiddurchlässig ist, vorzugweise ein perforiertes Geflecht aufweist, und wobei das Griffelement u-förmig mit zwei freien Schenkeln und einem diese miteinander verbindenden Griffstück ausgebildet und mit den freien Schenkeln am Sterilbehälterunterteil angelenkt ist, wobei die Deckelebene in oder unterhalb einer durch einen Rand des Sterilbehälterunterteils aufgespannten virtuellen Ebene liegt.

Es ist ein besonderer Vorteil der Erfindung, dass die Griffmulde / der Aufnahmebereich gegenüber der Deckelebene oder Hauptebene des Sterilbehälterdeckels abgesenkt ist. Der Aufnahmebereich bzw. die Griffmulde nimmt das Griffelement der Siebschale so auf, dass es im geschlossenen Zustand unterhalb der Deckeloberfläche, insbesondere vollständig unterhalb der Deckeloberfläche, positioniert ist und so nicht über die Deckelebene bzw. die Deckeloberfläche nach oben, also in die vom Aufnahmeraum abgewandte Richtung, hervorragt. Bei einem Stapeln mehrerer solcher verschlossener und verriegelter Behälter befindet sich das Griffelement des Sterilbehälterunterteils eines unteren Sterilbehälters daher unterhalb der Aufstandsfläche eines oberen Sterilbehälterunterteils, so dass der obere Sterilbehälter im Wesentlichen vollflächig auf der Deckelebene des Sterilbehälterdeckels des unteren Sterilbehälters zur Anlage / Auflage kommt. Außerdem gewährleistet die Erfindung, dass das Griffelement des Sterilbehälterunterteils in der Verriegelungsposition gut erreichbar und einfach in die Trageposition zu verbringen ist. Bei in der Trageposition befindlichem Griffelement kann der mit dem Sterilbehälterdeckel verschlossene Sterilbehälter außerdem besonders sicher getragen werden, auch im beladenen Zustand mit einem relativ hohen Gewicht, da das Griffelement am Sterilbehälterunterteil angeordnet ist. Das Verbringen des Griffelements aus der Verriegelungsposition in die Trageposition und umgekehrt kann in vorteilhafter Weise erfolgen, ohne dass der Deckel geöffnet werden muss bzw. geöffnet werden kann. Insgesamt vereint die Erfindung gleich eine Mehrzahl an Vorteilen, die sich positiv in einem ersten Aspekt auf die Lagereignung / Stapelbarkeit, in einem zweiten Aspekt auf die Tragbarkeit und in einem dritten Aspekt auf die Wahrung von Sterilitätsbedingungen auswirken.

Die der Erfindung zugrundeliegende Aufgabe wird außerdem gelöst durch ein Verfahren zum Herstellen eines Sterilbehälters mit einem einen Aufnahmeraum ausbildenden Sterilbehälterunterteil und einem Sterilbehälterdeckel zum Verschließen des Aufnahmeraums, insbesondere eines Sterilbehälters nach der vorliegenden Beschreibung oder nach einem der angehängten Ansprüche, wobei in dem Sterilbehälterdeckel ein Aufnahmebereich zur vollständigen Aufnahme des Griffelements darin in einer Verriegelungsposition ausgebildet ist, aufweisend die folgenden Schritte:
- Bereitstellen eines Bleches, insbesondere aus einem rostfreien Stahlmaterial,
- Einbringen von Schlitzen in das Blech, um zur Ausbildung der Griffmulde einen zur Verformung vorgesehenen Bereich zwischen der Deckelebene und der Griffmulde bereitzustellen,
- Ausbilden der Griffmulde für das Griffelement durch Verformen des mit Schlitzen versehenen Bereichs des Blechs in die zum Aufnahmeraum des Sterilbehälterunterteils weisende Richtung.

Vorteilhafte Ausführungsformen der Erfindung sind in den Unteransprüchen beansprucht und werden nachfolgend näher erläutert.

Der Sterilbehälter besitzt vorzugsweise einen im Wesentlichen rechteckigen Querschnitt. Jeweils eine Griffmulde ist auf zwei einander gegenüberliegenden Seiten, vorzugsweise den Kurzseiten, des Behälterdeckels ausgebildet. Die sich gegenüberliegenden Griffmulden sind vorzugsweise spiegelsymmetrisch zueinander ausgebildet und angeordnet. Die Form / Außenform der Griffmulde entspricht vorzugsweise der Außenform des Griffelements.

Eine Ausführungsform ist dadurch gekennzeichnet, dass der Sterilbehälterdeckel eine im Wesentlichen ebene Deckelfläche aufweist und die Vertiefung relativ zur Deckelfläche nach Innen in den Aufnahmeraum hineinragt. Eine solche ebene Deckelfläche ist in besonders vorteilhafter Weise fertigungs- und reinigungsfreundlich und bietet eine besonders große Auflagerfläche für einen aufgestapelten weiteren Sterilbehälter. Vorzugsweise liegt die Deckelfläche in einer Ebene mit einer durch den Rand des Sterilbehälterunterteils ausgebildeten virtuellen Ebene. Dies ermöglicht ein möglichst großes Behältervolumen. Bei einer weiteren Ausführungsform kann die Deckelfläche /Deckelebene unterhalb der durch den Rand des Sterilbehälterunterteils aufgespannten virtuellen Ebene liegen, also auf der dem Aufnahmeraum zugewandten Seite. Auf diese Weise kann ein die Oberseite der Deckelebene (die vom Aufnahmeraum abgewandte Seite der Deckelebene) umgebender Rand am ansonsten möglichst ebenen Deckel ausgebildet sein, der als Lagesicherung und Positionierungshilfe für auf den Deckel gestapelte weitere Sterilbehälter dient.

Nach einer weiteren Ausführungsform ist der Aufnahmebereich ausgebildet, indem die Deckelfläche nach Innen in den Aufnahmeraum verformt ist. Alternativ kann der Aufnahmebereich durch ein an der Deckelfläche befestigtes, insbesondere angeschweißtes, Blechformteil gebildet sein. Hierbei kann die Verbindung zwischen der Deckelfläche und dem angeschweißten Blechformteil durch Verbindungslaschen realisiert werden, die an ihren jeweiligen Enden entweder mit der Deckelfläche oder dem Blechformteil verschweißt werden. Ferner lassen die Verbindungslaschen Aussparungen im Übergangsbereich zwischen der Deckelfläche und dem Blechformteil zu, wodurch der Aufnahmebereich bei einer Reinigung besonders gut von einem Reinigungsfluid umspült werden kann, indem die Aussparungen im Aufnahmebereich von Reinigungsfluid durchspült werden.

Nach einer weiteren Ausführungsform kann das Griffelement u-förmig mit zwei freien Schenkeln und einem diese miteinander verbindenden Griffstück ausgebildet sein. Es ist vorzugsweise mit den freien Schenkeln am Sterilbehälter, insbesondere an einem oberen Rand des Sterilbehälters, angelenkt. Infolge der Anlenkung kann es besonders einfach in die Verriegelungsposition und die Tragposition verbracht / positioniert werden. Durch eine Anlenkung am oberen Rand des Sterilbehälterunterteils wird der Vorteil bewirkt, dass der Aufnahmebereiche / die Griffmulde nicht besonders tief ausgebildet werden muss, um eine komplette Aufnahme und gute Erreichbarkeit des Griffelements für einen Nutzer sicherzustellen. Außerdem wird die Größe / Höhe des Aufnahmeraums des Sterilbehälters nur möglichst wenig beschränkt. Bei einem solchen u-förmigen Griffelement besitzt die Griffmulde eine im Wesentlichen u-förmige Umfangsform. Anders ausgedrückt ist der Boden der Griffmulde eine der Form des Griffelements entsprechende Umfangsform, hier also eine im Wesentlichen u-förmige Umfangsform, und ist außerdem vorzugsweise vollflächig eben ausgebildet.

Vorzugsweise sind die beiden freien Schenkel und das Griffstück eben ausgebildet. In der Verriegelungsposition sind sie vorzugsweise parallel zur Deckelfläche ausgerichtet und/oder vollständig auf der dem Aufnahmeraum zugewandten Seite der Deckelfläche angeordnet.

Eine weitere Ausführungsform der Erfindung ist dadurch gekennzeichnet, dass der Sterilbehälterdeckel ein aus rostfreiem Stahlblech hergestellter perforierter Sterilbehälterdeckel ist. Alternativ oder zusätzlich kann das Sterilbehälterunterteil grundsätzlich ein aus rostfreiem Stahlblech hergestelltes perforiertes Sterilbehälterunterteil sein. Auf diese Weise stellt die Erfindung einen Sterilbehälter in Form eines Siebschalenbehälters zur Verfügung. Die Herstellung der Griffmulde kann auf unterschiedliche Arten und Weisen erfolgen, zum Beispiel in Form von separaten Elementen, die am Deckel adaptiert sind, oder direkt per Verformung aus dem DeckelBlech. Die die Griffmulde ausbildende Vertiefung kann insbesondere durch ein an der Deckelfläche befestigtes, insbesondere angeschweißtes, Blechformteil gebildet sein. Alternativ und wie schon zuvor dargelegt kann die die Griffmulde ausbildende Vertiefung durch einen verformten Bereich der Deckelebene ausgebildet sein. Beide Varianten ermöglichen eine einfache Fertigung eines gut zu reinigenden Deckels.

Eine besonders gut zu reinigende Griffmulde kann bewirkt werden, indem der verformte Bereich durch in die Deckelebene eingebrachte zueinander parallele Schlitze oder Durchbrüche gebildet ist, die nach Verformung eine rautenförmige Gitterstruktur ausbilden. Auf diese Weise wird ein Deckel zur Verfügung gestellt, dessen Griffmulde bei einer Reinigung besonders gut von einem Reinigungsfluid gespült werden kann, indem die Durchbrüche an der Griffmulde bzw. im Bereich der Griffmulde von Reinigungsfluid durchspült werden. Solche Abschnitte des Deckels / des Deckelblechs, die im Fertigungsprozess verformt werden, sind bei dieser Ausführungsform quasi nach dem Prinzip eines "Streckmetalls" ausgeführt. Das bedeutet, dass an bzw. in dem Deckelblech Durchgangsöffnungen ausgebildet werden, insbesondere schlitzförmige Durchbrüche, die zum Beispiel durch einen Stanzvorgang erzeugt werden bzw. vorliegen, die sich durch Streckung während der Verformung des Deckelmaterials zur Griffmulde zu beispielsweise rautenförmige Öffnungen verformen.

Eine weitere Ausführungsform des Sterilbehälters ist dadurch gekennzeichnet, dass in dem Aufnahmebereich eine Raststruktur zur Verrastung mit dem Griffelement in der Verriegelungsstellung ausgebildet ist. Diese kann durch eine entsprechende Formgebung des Deckelmaterials im Bereich der Griffmulde ausgebildet sein. Eine besonders einfach durch Verformung herzustellende Griffmulde kann erzielt werden, indem die Raststruktur durch ein an dem Sterilbehälterdeckel, insbesondere in der Griffmulde, festgelegtes Federelement, insbesondere aus Kunststoff / Spritzguss ausgebildet ist. Es ist besonders elegant, wenn das Rastelement Befestigungsvorsprünge aufweist, die in passend geformte Befestigungsausnehmungen im Deckelmaterial eingreifen und damit vorzugsweise verrasten. Die Befestigungsausnehmungen können beispielsweise durch in das Deckelmaterial eingebrachte, insbesondere ausgestanzte, Durchgangsöffnungen gebildet sein.

An dieser Stelle sei darauf hingewiesen, dass die vorstehend genannte Raststruktur natürlich auch anderweitig bereitgestellt sein kann. So kann diese alternativ zu der vorstehenden Ausführungsform auch aus einem Kunststoff, beispielsweise einem Elastomer, bevorzugt einem Silikonmaterial gefertigt/ausgebildet sein. Dieses kann dann als separat hergestelltes Bauteil im Bereich der Griffmulde am Deckel(material) montiert sein. Außerdem kann die Raststruktur insbesondere dann, wenn sie als separates Kunststoffelement bereitgestellt wird, ein elastischer Formkörper sein.

Bei weiteren Ausführungsformen des erfindungsgemäßen Verfahrens kann außerdem vorgesehen sein, dass in das Blechmaterial für den Sterilbehälterdeckel Perforationen eingebracht, insbesondere ausgestanzt werden, die bei einer Umformung von Abschnitten des Deckelmaterials zu Griffmulden nicht verformt werden. Bei einer weiteren Ausführungsform des Verfahrens kann das Blechmaterial nach dem Einbringen aller Perforationen einem Waschvorgang unterzogen werden, dem sich optional ein Richtvorgang zum Beispiel in Form einer Walzbearbeitung anschließt. Bei einer weiteren Ausführungsform kann das Blechmaterial anschließend einem Schleif- und/oder Entgratungsprozess unterzogen werden. Optional kann ein weiterer Waschvorgang durchgeführt werden. Erst nach diesen Verfahrensschritten erfolgt das Ausformen / Verformen des Materials zur Griffmulde bzw. zu den Griffmulden, insbesondere durch Pressen. Optional kann nachfolgend ein vorzugsweise vollumfänglich umlaufender Rand als Begrenzung für auf dem Sterilbehälterdeckel gestapelte weitere erfindungsgemäße Behälter ausgebildet werden, wiederum vorzugsweise durch Pressen. Es ist besonders effektiv und günstig, wenn das Formen der Griffmulde / Griffmulden und optional des Stapelrand in einem einzigen Prägewerkzeug bzw. einem Prozessschritt realisiert werden.

Man kann auch sagen, dass die Erfindung einen aus rostfreiem Stahlblech hergestellten perforierten Sterilbehälterdeckel zur Verfügung stellt, der die Griffe der dazugehörigen Siebschale bzw. des Sterilbehälters stirnseitig in Griffmulden aufnimmt, die gegenüber der Hauptfläche so abgesenkt sind, dass die Griffe (inkl. optionaler Verschlusselemente) im geschlossenen Zustand unterhalb der Deckeloberfläche verbleiben. Auf diese Weise kann mittels der Erfindung ein Sterilbehälter / eine Siebschale zur Verfügung gestellt werden, bei dem bzw. der trotz Verwendung eines Sterilbehälterdeckels die Griffelemente für einen Nutzer gut erreichbar sind, der Sterilbehälter bzw. die Siebschale an den Griffelementen ( und nicht am Deckel) getragen werden kann und mehrere Sterilbehälter / Siebschalen besonders stabil aufeinander gestapelt werden können.

Weitere Merkmale und Vorteile der vorliegenden Erfindung ergeben sich aus der folgenden beispielhaften und nicht beschränkenden Beschreibung der Figuren. Diese sind lediglich schematischer Natur und dienen nur dem Verständnis der Erfindung. Dabei zeigen:
Fig. 1 eine perspektivische Ansicht eines Sterilbehälters nach einer ersten Ausführungsform der Erfindung,
Fig. 2 in einer perspektivischen Ansicht einen Abschnitt eines Sterilbehälters nach einer weiteren Ausführungsform der Erfindung,
Fig. 3 in einer perspektivischen Ansicht einen Abschnitt des Sterilbehälters der Figur 1,
Fig. 4 in einer perspektivischen Ansicht einen Teil zweier erfindungsgemäßer, aufeinandergestapelter Sterilbehälter,
Fig. 5 in einer perspektivischen Ansicht einen Teil eines Stanzblechs für einen Sterilbehälterdeckel eines erfindungsgemäßen Sterilbehälters und
Fig. 6 in einer perspektivischen Ansicht den Teil eines Stanzblechs der Figur 4 mit ausgeformten Griffmulden.

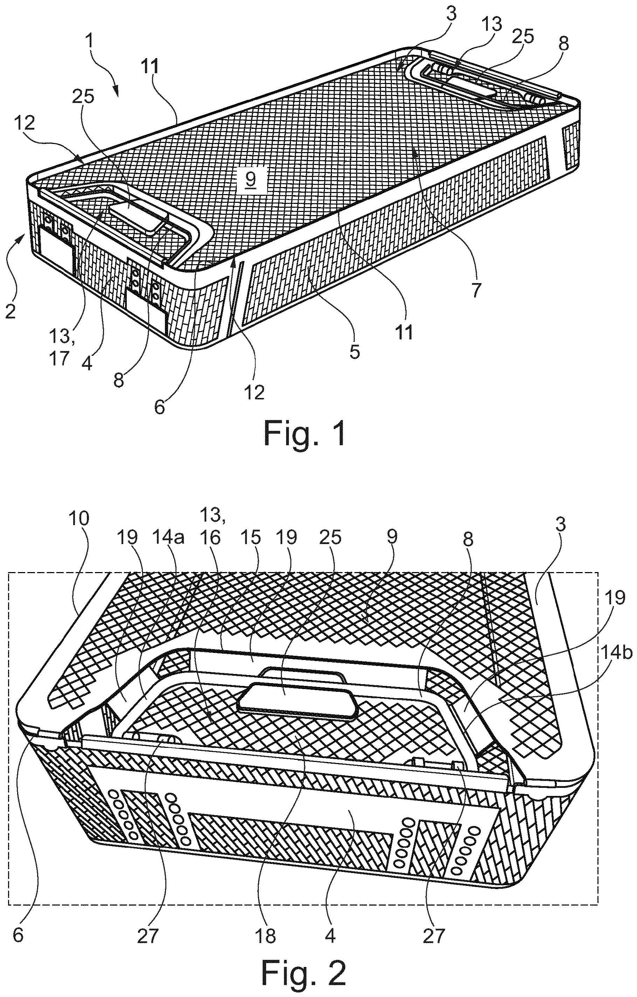

Figur 1 zeigt einen Sterilbehälter 1 nach einer Ausführungsform der Erfindung in einer perspektivischen Ansicht. Der Sterilbehälter 1 ist als Siebschale 1 ausgebildet, besitzt ein Sterilbehälterunterteil 2 und einen Sterilbehälterdeckel 3 und dient einer Aufnahme von sterilisiertem oder zu sterilisierendem medizinischem Gut, das in den Figuren nicht dargestellt ist. Das Sterilbehälterunterteil 2 weist einen Boden (in den Figuren nicht zu erkennen), zwei daran angeordnete Kurzseitenwände 4 sowie zwei Langseitenwände 5 auf. Die Kurzseitenwände 4 und die Langseitenwände 5 bilden gemeinsam einen umlaufenden Behälterunterteilrand 6 aus und umgeben einen Aufnahmeraum 7 für das medizinische Gut. Sowohl das Sterilbehälterunterteil 2 als auch der Sterilbehälterdeckel 2 sind jeweils aus einem rostfreien, perforierten Stahlblech hergestellt.

Der Sterilbehälterdeckel 3 ist auf dem umlaufenden Rand 6 lösbar angeordnet und dient dazu, den Aufnahmeraum 7 derart zu verschließen, dass einerseits in darin befindliches Gut vor Beschädigungen von außen geschützt ist und andererseits das darin aufgenommene Gut nicht versehentlich aus dem Aufnahmeraum 7 herausfallen kann.

Am Sterilbehälterunterteil 2 ist an beiden Kurzseitenwänden 4 jeweils ein Griffelement 8 gelenkig angeordnet. Dieses ist zwischen einer vom Sterilbehälterdeckel 3 abragenden Trageposition (in den Figuren nicht dargestellt) und einer den Sterilbehälterdeckel 3 mit dem Sterilbehälterunterteil 2 verriegelnden Verriegelungsposition (in den Figuren dargestellt) positionierbar. Das Griffelement 8 ist im Wesentlichen u-förmig mit zwei freien Schenkeln 14a und 14b und einem diese miteinander verbindenden Griffstück 15 ausgebildet. Es ist mit seinen freien Schenkeln 14a, 14b in Schwenklageraufnahmen 27 am oberen Rand 6 des Sterilbehälterunterteils 2 angelenkt.

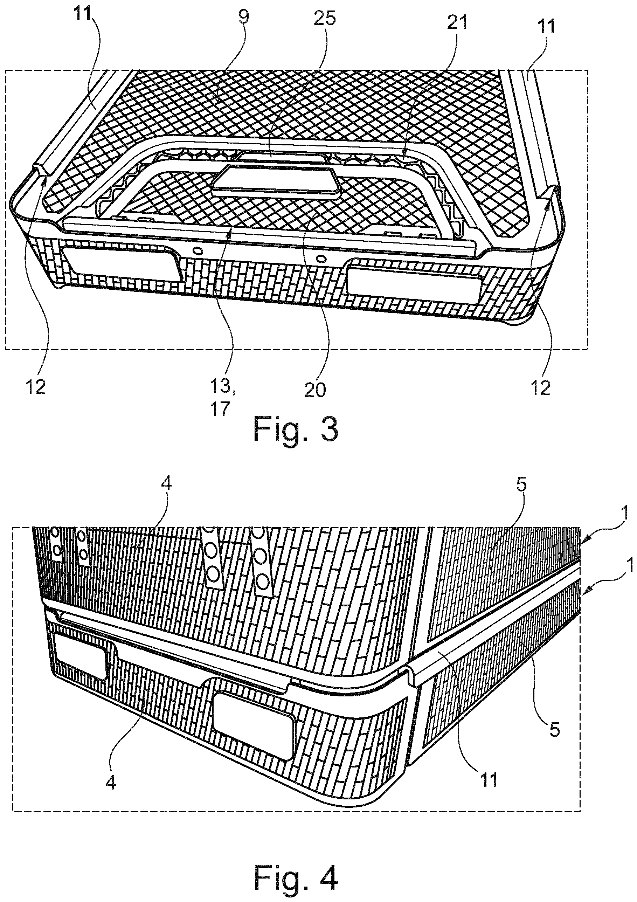

Der Sterilbehälterdeckel 3 ist als Blechstanzformteil ausgebildet. Er besitzt eine im Wesentlichen eben ausgebildete Deckelfläche 9, die quasi der Blechfläche im unverformten Zustand entspricht. Die Deckelfläche 9 bildet eine Aufstandsfläche oder Auflagerfläche zum Aufstapeln eines weiteren Sterilbehälters 1 aus (siehe in Figur 4). Bei der in Figur 2 gezeigten Ausführungsform befindet sich die Deckelfläche 9 bei auf den Behälterrand 6 aufgesetztem Behälterdeckel 3 quasi in der durch den Behälterrand 6 aufgespannten Ebene bzw. knapp darüber. Die Deckelfläche 9 ist bei dieser Ausführungsform vom einem teilweise umlaufenden Deckelrand 10 umgeben. Dieser umgreift den Behälterrand 6 und sorgt derart für einen lagebestimmten Sitz des Behälterdeckels 3 auf dem Behälterunterteil 2.

Die in den Figuren 1 und 3 gezeigte Ausführungsform unterscheidet sich von der in Figur 2 gezeigten Ausführungsform darin, dass die Deckelfläche 9 im Wesentlichen vollständig aus der von dem Behälterrand 6 aufgespannten Ebene in Richtung des Aufnahmeraums 7 versetzt ist. Dies wird dadurch bewirkt, dass die Deckelebene 9 von einem nach oben, also in die vom Aufnahmeraum 7 abgewandte Richtung, vorstehenden Deckelrand 11 umgeben ist, dessen dem Behälterrand 6 zugewandte Innenseite eine Aufnahmenut 12 für den Behälterrand 6 ausbildet.

In dem Sterilbehälterdeckel 3, genauer gesagt in dessen Deckelfläche 9, ist beiderseits jeweils eine Griffmulde 13 ausgebildet. Wie insbesondere in den Figuren 1, 2 und 3 gezeigt ist, ist das an der entsprechenden Kurzseitenwand 4 angelenkte und in der Verriegelungsposition befindliche Griffelement 8 vollständig in der jeweiligen Griffmulde 13 aufgenommen, derart, dass kein Teil des Griffelements 8 über die Deckelfläche 9 nach außen hervorsteht.

Bei allen in den Figuren dargestellten Ausführungsformen ist die Deckelfläche 9 im Wesentlichen eben und die Griffmulde 13 in Form einer Vertiefung relativ zur Deckelfläche 9 nach Innen in den Aufnahmeraum 7 hineinragend ausgebildet.

Ein weiterer Unterschied zwischen den Ausführungsformen der Figuren 1 und 3 einerseits und der Figur 2 andererseits ist, dass bei der Ausführungsform der Figur 2 die Griffmulden 13 jeweils durch ein an die Deckelfläche 9 angeschweißtes Blechformteil 16 gebildet ist, während bei der Ausführungsform der Figuren 1 und 3 die Griffmulden 13 jeweils durch einen verformten Abschnitt 17 gebildet sind. Figur 2 zeigt, dass das Blechformteil 16 einen perforierten Griffmuldenboden 18 und daran angeordnete Verbindungslaschen 19 aufweist, die mit der Deckelfläche 9 verschweißt sind. Der verformte Abschnitt 17 hingegen weist einen ebenfalls perforierten Griffmuldenboden 20 und eine sich zwischen diesem und der Deckelfläche 9 erstreckende ebenfalls perforierte Griffmuldenwand 21 auf.

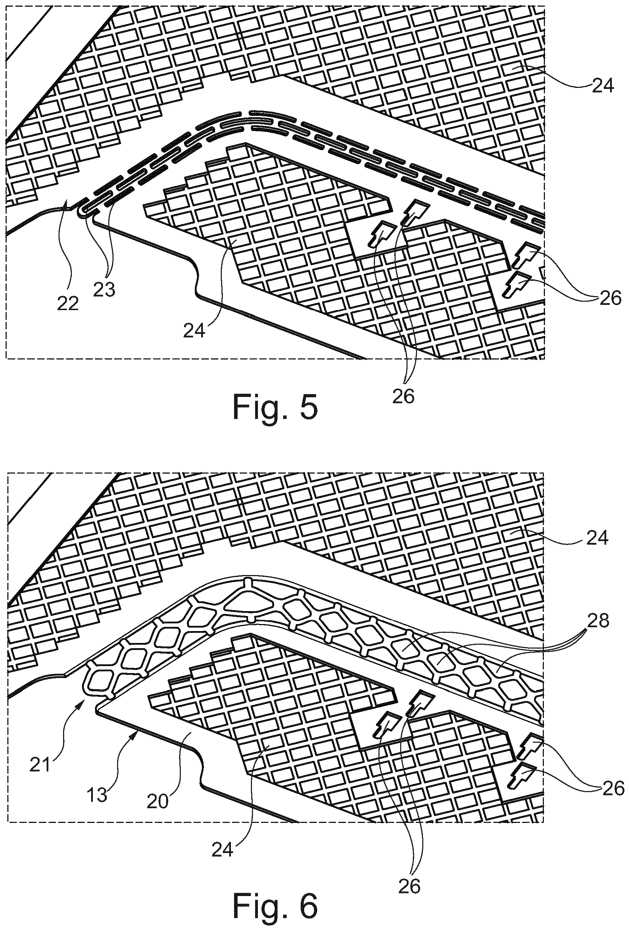

Im Zusammenhang mit der Ausführungsform der Figur 2 verdeutlichen die Figuren 5 und 6 die Herstellung der Griffmulde 13 aus einem ebenen Blechmaterial 22. Dabei zeigt Figur 5 das Blechmaterial 22 in einem noch unverformten Zustand, während Figur 6 die durch Verformung ausgebildete Griffmulde 13 darstellt. Das Blechmaterial 22 ist mit ersten Perforationen 24 versehen, und zwar im Bereich der späteren Deckelfläche 9 und im Bereich des späteren Griffmuldenbodens 20. Zwischen diesen beiden Bereichen ist ein Abschnitt mit im unverformten Zustand (Figur 5) schlitzförmigen Durchgangsöffnungen 23 ausgebildet. Im Rahmen der Umformung aus dem in Figur 5 gezeigten Zustand in den in Figur 6 gezeigten, die Griffmulde 13 ausbildenden Zustand weiten sich die schlitzförmigen Durchgangsöffnungen 23 zu im Wesentlichen rautenförmigen Perforationen 28 auf.

Unabhängig von der jeweiligen Ausführungsform ist in der Griffmulde 13 jeweils eine Raststruktur 25 zur Verrastung mit dem in der Verriegelungsstellung befindlichen Griffelement 8, genauer mit dessen Griffstück 15 ausgebildet. Die Raststruktur 25 ist vorzugsweise ein Kunststoffteil 25, das in der Griffmulde 13 an dem Sterilbehälterdeckel 3 befestigt ist. Zu diesem Zweck weist es in den Figuren nicht gezeigte Befestigungselemente auf, die in passend geformte Befestigungsausnehmungen 26 im Griffmuldenboden 18, 20 eingreifen und damit verrasten. Die Befestigungsausnehmungen 26 sind hier in Form von durch in das Deckelmaterial eingebrachte und ausgestanzte Durchgangsöffnungen 26 gebildet.

### Bezugszeichenliste

- 1: Sterilbehälter, Siebschale
- 2: Sterilbehälterunterteil
- 3: Sterilbehälterdeckel
- 4: Kurzseitenwand
- 5: Langseitenwand
- 6: Behälterunterteilrand, umlaufender Rand
- 7: Aufnahmeraum
- 8: Griffelement
- 9: Deckelfläche
- 10: Deckelrand
- 11: Deckelrand
- 12: Aufnahmenut
- 13: Griffmulde
- 14a: freier Schenkel
- 14b: freier Schenkel
- 15: Griffstück
- 16: Blechformteil
- 17: verformter Abschnitt
- 18: Griffmuldenboden
- 19: Verbindungslasche
- 20: Griffmuldenboden
- 21: perforierte Griffmuldenwand
- 22: Blechmaterial
- 23: schlitzförmige Durchgangsöffnungen
- 24: rautenförmige Perforationen
- 25: Raststruktur
- 26: Befestigungsausnehmungen, Durchgangsöffnungen
- 27: Schwenklageraufnahmen
- 28: rautenförmige Perforationen

## Patentansprüche

1. Sterilbehälter (1) zur Aufnahme von gereinigtem oder sterilisiertem oder zu reinigendem oder zu sterilisierendem medizinischem Gut, mit einem einen Aufnahmeraum (7) für das Gut ausbildenden Sterilbehälterunterteil (2) und einem an dem Sterilbehälterunterteil (2) lösbar anordbaren Sterilbehälterdeckel (3) zum Verschließen des Aufnahmeraums (7), wobei am Sterilbehälterunterteil (2) zumindest ein Griffelement (8) angeordnet ist, das zwischen einer vom Sterilbehälterdeckel (3) abragenden Trageposition und einer den Sterilbehälterdeckel (3) mit dem Sterilbehälterunterteil (2) verriegelnden Verriegelungsposition positionierbar ist, wobei in dem Sterilbehälterdeckel (3) ein Aufnahmebereich (13) zur Aufnahme des Griffelements (8) darin in der Verriegelungsposition ausgebildet ist,
wobei
der Aufnahmebereich (13) als Griffmulde (13) in Form einer gegenüber der Deckelebene (9) in den Aufnahmeraum (7) hineinragenden Vertiefung zur vollständigen Aufnahme des Griffelements (8) darin ausgebildet ist, und
wodurch sich zumindest ein Steg zwischen dem Sterilbehälterdeckel (3) und dem Aufnahmebereich (13) als Griffmulde (13) bildet,
wobei
der Steg zumindest abschnittsweise fluiddurchlässig ist, vorzugsweise ein perforiertes Geflecht aufweist, **dadurch gekennzeichnet, dass** das Griffelement (8) u-förmig mit zwei freien Schenkeln (14a, 14b) und einem diese miteinander verbindenden Griffstück (15) ausgebildet und mit den freien Schenkeln (14a, 14b) am Sterilbehälterunterteil (2) angelenkt ist,
wobei
die Deckelebene (9) in oder unterhalb einer durch einen Rand (6) des Sterilbehälterunterteils (2) aufgespannten virtuellen Ebene liegt.

2. Sterilbehälter (1) nach Anspruch 1, **dadurch gekennzeichnet, dass** die beiden freien Schenkel (14a, 14b) und das Griffstück (15) eben ausgebildet sind und in der Verriegelungsposition parallel zur Deckelebene (9) ausgerichtet sind und/oder in der Verriegelungsposition vollständig auf der dem Aufnahmeraum (7) zugewandten Seite der Deckelebene (9) angeordnet sind.

3. Sterilbehälter (1) nach Anspruch 2, **dadurch gekennzeichnet, dass** der Sterilbehälterdeckel (3) eine im Wesentlichen ebene Deckelebene (9) aufweist und die Vertiefung der Griffmulde (13) relativ zur Deckelebene (9) nach Innen in den Aufnahmeraum (7) hineinragt.

4. Sterilbehälter (1) nach Anspruch 3, **dadurch gekennzeichnet, dass** die Griffmulde (13) ausgebildet ist, indem die Deckelebene (9) nach Innen in den Aufnahmeraum (7) verformt ist.

5. Sterilbehälter (1) nach einem der vorstehenden Ansprüche, **dadurch**
**gekennzeichnet, dass** der Sterilbehälterdeckel (3) ein aus rostfreiem Stahlblech hergestellter perforierter Sterilbehälterdeckel (3) ist.

6. Sterilbehälter (1) nach einem der vorstehenden Ansprüche, **dadurch**
**gekennzeichnet, dass** die die Griffmulde (13) ausbildende Vertiefung durch ein an der Deckelebene (9) befestigtes Blechformteil (16) gebildet ist, oder dass die die Griffmulde (13) ausbildende Vertiefung durch einen verformten Bereich (17) der Deckelebene (9) ausgebildet ist.

7. Sterilbehälter (1) nach dem vorstehenden Anspruch, **dadurch gekennzeichnet, dass** der verformte Bereich (17) durch in die Deckelebene (9) eingebrachte zueinander parallele Schlitze (23) oder Durchbrüche (23) gebildet ist, die nach Verformung eine rautenförmige Gitterstruktur ausbilden.

8. Sterilbehälter (1) nach einem der vorstehenden Ansprüche, **dadurch**
**gekennzeichnet, dass** in der Griffmulde (13) eine Raststruktur (25) zur Verrastung mit dem Griffelement (8) in der Verriegelungsstellung ausgebildet ist.

9. Sterilbehälter (1) nach dem vorstehenden Anspruch, **dadurch gekennzeichnet, dass** die Raststruktur (25) durch ein an dem Sterilbehälterdeckel (3) festgelegtes Federelement (25) ausgebildet ist.

10. Verfahren zum Herstellen eines Sterilbehälters (1) nach einem der vorstehenden Ansprüche mit einem einen Aufnahmeraum (7) ausbildenden Sterilbehälterunterteil (2) und einem Sterilbehälterdeckel (3) zum Verschließen des Aufnahmeraums (7), wobei in dem Sterilbehälterdeckel (3) eine Griffmulde (13) zur vollständigen Aufnahme des Griffelements (8) darin in einer Verriegelungsposition ausgebildet ist, aufweisend die folgenden Schritte:
- Bereitstellen eines Bleches,
- Einbringen von Schlitzen (23) in das Blech, um zur Ausbildung der Griffmulde (13) einen zur Verformung vorgesehenen Bereich (17) zwischen der Deckelebene (9) und der Griffmulde (13) bereitzustellen,
- Ausbilden der Griffmulde (13) für das Griffelement (8) durch Verformen des mit Schlitzen (23) versehenen Bereichs (17) des Blechs in die zum Aufnahmeraum (7) des Sterilbehälterunterteils (2) weisende Richtung.

## Claims

1. A sterile container (1) for receiving cleaned or sterilized medical goods or medical goods to be cleaned or sterilized, with a lower part (2) of the sterile container forming a receiving space (7) for the goods and a sterile-container lid (3) which is detachably arrangeable on the lower part (2) of the sterile container for closing the receiving space (7), wherein at least one handle element (8) is arranged on the lower part (2) of the sterile container, said handle element (8) being positionable between a carrying position projecting from the sterile-container lid (3) and a locking position locking the sterile-container lid (3) to the lower part (2) of the sterile container, wherein a receiving region (13) is formed in the sterile-container lid (3) for receiving the handle element (8) therein in the locking position,
wherein
the receiving region (13) is formed as a recessed grip (13) in the form of a depression projecting into the receiving space (7) relative to the lid plane (9) for completely receiving the handle element (8) therein, and
whereby at least one web is formed between the sterile-container lid (3) and the receiving region (13) as a recessed grip (13),
wherein
the web is fluid-permeable at least in sections, preferably comprises a perforated mesh, **characterized in that**
the handle element (8) is U-shaped with two free legs (14a, 14b) and a handle piece (15) connecting them to each other and is hinged with the free legs (14a, 14b) to the lower part (2) of the sterile container
wherein the lid plane (9) is in or below a virtual plane defined by an edge (6) of the lower part (2) of the sterile container.

2. The sterile container (1) according to claim 1, **characterized in that** the two free legs (14a, 14b) and the handle piece (15) are plane and, in the locking position, are oriented parallel to the lid surface (9) and/or, in the locking position, are arranged completely on the side of the lid surface (9) facing the receiving space (7).

3. The sterile container (1) according to claim 2, **characterized in that** the sterile-container lid (3) has a substantially plane lid plane (9) and the depression of the recessed grip (13) projects inward into the receiving space (7) relative to the lid plane (9).

4. The sterile container (1) according to claim 3, **characterized in that** the recessed grip (13) is formed by deforming the lid plane (9) inward into the receiving space (7).

5. The sterile container (1) according to one of the preceding claims, **characterized in that** the sterile-container lid (3) is a perforated sterile-container lid (3) made of a stainless steel sheet.

6. The sterile container (1) according to one of the preceding claims, **characterized in that** the depression forming the recessed grip (13) is formed by a sheet metal part (16) fixed to the lid plane (9), or that the depression forming the recessed grip (13) is formed by a deformed region (17) of the lid plane (9).

7. The sterile container (1) according to the preceding claim, **characterized in that** the deformed region (17) is formed by slots (23) or openings (23) formed in the lid plane (9) that are parallel to each other and form a rhombic grid structure after deformation.

8. The sterile container (1) according to one of the preceding claims, **characterized in that** a latching structure (25) is formed in the recessed grip (13) for latching with the handle element (8) in the locked position.

9. The sterile container (1) according to the preceding claim, **characterized in that** the latching structure (25) is formed by a spring element (25) fixed to the sterile-container lid (3).

10. A method of manufacturing a sterile container (1) according to one of the preceding claims, having a lower part (2) of the sterile container forming a receiving space (7) and a sterile-container lid (3) for closing the receiving space (7), wherein a recessed grip (13) is formed in the sterile-container lid (3) for completely receiving the handle element (8) therein in a locking position, comprising the following steps:
- providing a metal sheet,
- making slots (23) in the sheet metal to provide a region (17) for deformation between the lid plane (9) and the recessed grip (13) to form the recessed grip (13),
- forming the recessed grip (13) for the handle element (8) by deforming the region (17) of the sheet metal provided with slots (23) in the direction facing the receiving space (7) of the lower part (2) of the sterile container.

## Revendications

1. Récipient de stérilisation (1) pour la réception de produits médicaux nettoyés ou stérilisés ou à nettoyer ou à stériliser, avec une partie inférieure de récipient de stérilisation (2) formant un espace de réception (7) pour les produits et un couvercle de récipient de stérilisation (3) pouvant être agencé de manière amovible au niveau de la partie inférieure de récipient de stérilisation (2) pour la fermeture de l'espace de réception (7), dans lequel au moins un élément de préhension (8) est agencé au niveau de la partie inférieure de récipient de stérilisation (2), élément qui peut être positionné entre une position de portage dépassant du couvercle de récipient de stérilisation (3) et une position de verrouillage verrouillant le couvercle de récipient de stérilisation (3) avec la partie inférieure de récipient de stérilisation (2), dans lequel une zone de réception (13) est formée dans le couvercle de récipient de stérilisation (3) pour la réception de l'élément de préhension (8) à l'intérieur dans la position de verrouillage,
dans lequel
la zone de réception (13) est formée comme poignée encastrée (13) sous la forme d'un évidement pénétrant par rapport au plan de couvercle (9) dans l'espace de réception (7) pour la réception complète de l'élément de préhension (8) à l'intérieur, et selon lequel au moins une nervure se forme entre le couvercle de récipient de stérilisation (3) et la zone de réception (13) comme poignée encastrée (13),
dans lequel
la nervure est perméable au fluide au moins par sections, de préférence présente un treillis perforé, **caractérisé en ce que** l'élément de préhension (8) est formé en U avec deux branches libres (14a, 14b) et une pièce de préhension (15) reliant celles-ci l'une à l'autre et est articulé avec les branches libres (14a, 14b) à la partie inférieure de récipient de stérilisation (2),
dans lequel
le plan de couvercle (9) se trouve dans ou en dessous d'un plan virtuel serré par un bord (6) de la partie inférieure de récipient de stérilisation (2).

2. Récipient de stérilisation (1) selon la revendication 1, **caractérisé en ce que** les deux branches libres (14a, 14b) et la pièce de préhension (15) sont formées de manière plane et sont orientées dans la position de verrouillage parallèlement au plan de couvercle (9) et/ou sont complètement agencées dans la position de verrouillage sur le côté tourné vers l'espace de réception (7) du plan de couvercle (9).

3. Récipient de stérilisation (1) selon la revendication 2,
**caractérisé en ce que**
le couvercle de récipient de stérilisation (3) présente un plan de couvercle (9) sensiblement plan et l'évidement de la poignée encastrée (13) pénètre par rapport au plan de couvercle (9) vers l'intérieur dans l'espace de réception (7).

4. Récipient de stérilisation (1) selon la revendication 3,
**caractérisé en ce que**
la poignée encastrée (13) est formée en déformant le plan de couvercle (9) vers l'intérieur dans l'espace de réception (7).

5. Récipient de stérilisation (1) selon l'une quelconque des revendications précédentes, **caractérisé en ce que** le couvercle de récipient de stérilisation (3) est un couvercle de récipient de stérilisation (3) perforé fabriqué en tôle d'acier inoxydable.

6. Récipient de stérilisation (1) selon l'une quelconque des revendications précédentes, **caractérisé en ce que** l'évidement formant la poignée encastrée (13) est formé par une pièce usinée en tôle (16) fixée au plan de couvercle (9), ou **en ce que** l'évidement formant la poignée encastrée (13) est formé par une zone (17) déformée du plan de couvercle (9).

7. Récipient de stérilisation (1) selon la revendication précédente, **caractérisé en ce que** la zone (17) déformée est formée par des fentes (23) ou percées (23) parallèles les unes aux autres ménagées dans le plan de couvercle, qui forment après déformation une structure de grille en losange.

8. Récipient de stérilisation (1) selon l'une quelconque des revendications précédentes, **caractérisé en ce qu'**une structure d'encliquetage (25) est formée dans la poignée encastrée (13) pour l'encliquetage avec l'élément de préhension (8) dans la position de verrouillage.

9. Récipient de stérilisation (1) selon la revendication précédente, **caractérisé en ce que** la structure d'encliquetage (25) est formée par un élément de ressort (25) fixé au couvercle de récipient de stérilisation (3).

10. Procédé de fabrication d'un récipient de stérilisation (1) selon l'une quelconque des revendications précédentes avec une partie inférieure de récipient de stérilisation (2) formant un espace de réception (7) et un couvercle de récipient de stérilisation (3) pour la fermeture de l'espace de réception (7), dans lequel une poignée encastrée (13) est formée dans le couvercle de récipient de stérilisation (3) pour la réception complète de l'élément de préhension (8) à l'intérieur dans une position de verrouillage, présentant les étapes suivantes :
- la fourniture d'une tôle,
- le ménagement de fentes (23) dans la tôle afin de fournir, pour la formation de la poignée encastrée (13), une zone (17) prévue pour la déformation entre le plan de couvercle (9) et la poignée encastrée (13),
- la formation de la poignée encastrée (13) pour l'élément de préhension (8) par la déformation de la zone (17) pourvue de fentes (23) de la tôle dans le sens tourné vers l'espace de réception (7) de la partie inférieure de récipient de stérilisation (2).
